# EUROPÄISCHE PATENTSCHRIFT

(11) **EP 3 636 202 B1**
(45) Veröffentlichungstag und Bekanntmachung des Hinweises auf die Patenterteilung: **15.06.2022**
(21) Anmeldenummer: 18199458.3
(22) Anmeldetag: 09.10.2018
(51) Int. Cl.: A61C 1/08, A61B 6/03, A61B 6/14, G03B 42/02, A61B 90/00, A61B 34/20, A61B 34/10

(54) **DENTALES FUEHRUNGSSYSTEM ZUR ZAHNPRAEPARATION**
DENTAL GUIDING SYSTEM FOR DENTAL PREPARATION
SYSTÈME DE GUIDAGE DENTAIRE DESTINÉ À LA PRÉPARATION DES DENTS

(43) Veröffentlichungstag der Anmeldung: 15.04.2020
(73) Patentinhaber: Ivoclar Vivadent AG, 9494 Schaan (LI)
(72) Erfinder: FOLLONIER, Stéphane, 7324 Viters (CH); WACHTER, Wolfgang, 9494 Schaan (LI)
(74) Vertreter: Baldus, Oliver

(56) Entgegenhaltungen:
- WO-A1-2015/110859
- JP-B2- 5 043 145
- US-A1- 2015 310 668

## Beschreibung

Die vorliegende Erfindung betrifft ein dentales Führungssystem zur Zahnpräparation.

Bei der Formgebung von Zähnen im Mund eines Patienten kann es leicht zu Problemen kommen, weil Werkzeuge, wie beispielsweise Bohrer, nicht korrekt verwendet werden. Einmal abgetragenes Zahnmaterial ist dauerhaft entfernt und kann nicht wieder hinzugefügt werden. Grundsätzlich sollte die natürliche Zahnsubstanz soweit wie möglich erhalten bleiben.

Die Druckschrift WO 2015/110859 A1 betrifft ein Verfahren zum Führen eines Bohrers bei einer Implantatbehandlung. Die Position eines Bohrers wird erfasst und die relative Position des Bohrers zur beabsichtigten Position im Implantatplan verfolgt.

Die Druckschrift US 2015/310668 A1 betrifft ein Gerät zum Erhalten und Verarbeiten von dreidimensionalen Bildern (siehe Abstract). Bei einer Zahnbehandlung werden statische Röntgenbilder über dem echten Zahn überlagert.

Es ist daher die technische Aufgabe der vorliegenden Erfindung, die manuelle Formgebung von Zähnen im Mund eines Patienten zu vereinfachen und zu beschleunigen.

Diese Aufgabe wird durch Gegenstände nach den unabhängigen Ansprüchen gelöst. Vorteilhafte Ausführungsformen sind Gegenstand der abhängigen Ansprüche, der Beschreibung und der Figuren.

Gemäß einem ersten Aspekt wird die vorliegende Aufgabe durch ein dentales Führungssystem zur Zahnpräparation gelöst, mit einem elektronischen Erfassungssystem zum Erfassen einer dreidimensionalen Ist-Zahnform; einer Vergleichseinrichtung zum Ermitteln von räumlichen Abweichungsbereichen, an denen die ermittelte Ist-Zahnform von einer vorgegebenen Soll-Zahnform abweicht; und einer Datenbrille mit einer Anzeigeeinrichtung zum optischen Anzeigen der räumlichen Abweichungsbereiche. Das Erfassen einer dreidimensionalen Ist-Zahnform kann kontinuierlich und in Echtzeit während der Formgebung erfolgen. Durch das elektronische Erfassungssystem wird ein digitaler Datensatz erhalten, der die dreidimensionale Ist-Zahnform beschreibt und der von der Vergleichseinrichtung verarbeitet werden kann. Das Erfassen kann mittels eines optischen Verfahrens oder eines Röntgenverfahrens durchgeführt werden. Im Allgemeinen kann das elektronische Erfassungssystem jedes Erfassungssystem sein, mit dem sich ein digitaler Datensatz für die dreidimensionalen Ist-Zahnform erhalten lässt. Die Abweichungsbereiche werden direkt über den visuell wahrnehmbaren Zahn überlagert. Datenbrille mit der Anzeigeeinrichtung dient zum optischen Anzeigen der räumlichen Abweichungsbereiche über dem visuell wahrnehmbaren Zahn.

Durch das optische Hervorheben von Abweichungsbereichen durch die Datenbrille kann ein behandelnder Zahnarzt unmittelbar erkennen, welche räumlichen Bereiche des Zahns zu entfernen sind, damit die gewünschte Soll-Zahnform erhalten wird. Dadurch wird der technische Vorteil erreicht, dass ein unbeabsichtigtes Abtragen räumlicher Bereiche verhindert werden kann und möglichst viel natürliche Zahnsubstanz erhalten bleibt.

Die Ist-Zahnform ist beispielsweise die tatsächliche räumliche Form eines Zahnes, eines Kiefers, eines Bohrschablonenersatzes, einer Brücke oder einer Krone. Die Soll-Zahnform ist die räumliche Form des Zahnes, des Kiefers, des Bohrschablonenersatzes, der Brücke oder der Krone, die diese nach der manuellen Bearbeitung aufweisen sollen. Die Datenbrille ist ein als Brille getragenes elektronisches Gerät, mit dem einem Benutzer zusätzlich zur natürlichen visuellen Wahrnehmung weitere Informationen optisch angezeigt werden können. Die Datenbrille kann durch die Anzeigeeinrichtung zusätzliche Informationen auf einem Bild überlagern, das von dem Auge des Trägers wahrgenommen wird.

Die Anzeigeeinrichtung umfasst beispielsweise einen augennahen Bildschirm oder einen Projektor zur direkten Projektion auf der Netzhaut. Daneben können die Informationen auf einem externen Bildschirm angezeigt werden.

In einer technischen Ausführungsform des dentalen Führungssystems umfasst die Datenbrille das elektronische Erfassungssystem. Durch eine Integration des Erfassungssystems in die Datenbrille wird beispielsweise der technische Vorteil erreicht, dass sich der Aufbau des dentalen Führungssystems vereinfacht.

In einer weiteren technisch vorteilhaften Ausführungsform des dentalen Führungssystems umfasst das elektronische Erfassungssystem ein stereoskopisches Erfassungssystem mit einer ersten und einer zweiten Kamera oder ein stereoskopisches Erfassungssystem mit einer plenoptischen Kamera. Eine plenoptische Kamera, auch Lichtfeldkamera genannt, erfasst neben den üblichen zwei Bilddimensionen die Richtung einfallender Lichtstrahlen. Durch die zusätzliche Dimension enthalten plenoptische Aufnahmen Informationen über die Bildtiefe. Dadurch wird beispielsweise der technische Vorteil erreicht, dass die Ist-Zahnform mit geringem technischem Aufwand erfasst werden kann und sich das Erfassungssystem auf einfache Weise in eine Datenbrille integrieren lässt. Außerdem kann das Erfassen der dreidimensionalen Ist-Zahnform auch auf Basis mehrerer Bilder, mittels eines Streifenprojektionsverfahrens oder mittels einer Linsenrasterkamera erfolgen.

In einer weiteren technisch vorteilhaften Ausführungsform des dentalen Führungssystems umfasst das elektronische Erfassungssystem eine 3D-Kamera zum Erfassen einer dreidimensionalen Ist-Zahnform auf Basis einer Laufzeitmessung von Licht. Dadurch wird beispielsweise der technische Vorteil erreicht, dass sich die Zahnform auf schnelle Weise und mit einer kompakten Kamera ermitteln lässt.

In einer weiteren technischen Ausführungsform des dentalen Führungssystems ist die Vergleichseinrichtung ausgebildet, einen Restaurationsparameter auf Basis der erfassten Ist-Zahnform zu ermitteln. Der Restaurationsparameter ist beispielsweise ein Präparationswinkel, der angibt, um welchen Winkel die Präparation von der Längsachse abweicht. Im Allgemeinen kann es sich bei dem Restaurationsparameter jedoch um jeden Parameter handeln, der bei der Bearbeitung eine Rolle spielt. Dadurch wird beispielsweise der technische Vorteil erreicht, dass Restaurationsparameter überwacht werden können und sich die Formgebung des Zahnes weiter vereinfacht.

In einer weiteren technischen Ausführungsform des dentalen Führungssystems ist das Führungssystem ausgebildet, den Restaurationsparameter auf der Anzeigeeinrichtung der Datenbrille anzuzeigen. Dadurch wird beispielsweise der technische Vorteil erreicht, dass der Restaurationsparameter bei der manuellen Bearbeitung des Zahnes direkt berücksichtigt werden kann.

In einer weiteren technischen Ausführungsform des dentalen Führungssystems ist das elektronische Erfassungssystem ausgebildet, die dreidimensionale Ist-Zahnform auf Basis einer zeitlichen Serie von Zahnbildern zu ermitteln. Dadurch wird beispielsweise der technische Vorteil erreicht, dass sich die Zahnform auf einfache und genaue Weise ermitteln lässt. Jedes weitere Zahnbild der Serie erhöht die Genauigkeit der ermittelten Ist-Zahnform. Die Serie der Zahnbilder lässt sich beispielsweise zu einem (Teil-) Rundumbild des Zahnes zusammensetzen, das zur weiteren Rekonstruktion der Ist-Zahnform verwendet wird. Hierzu können spezielle Computeralgorithmen verwendet werden.

In einer weiteren technischen Ausführungsform des dentalen Führungssystems ist das elektronische Erfassungssystem ausgebildet, die Position eines Behandlungswerkzeuges zu erfassen. Dadurch wird beispielsweise der technische Vorteil erreicht, dass sich die Position des Behandlungswerkzeuges in Echtzeit verfolgen lässt. Auf Basis dieser Position kann das Behandlungswerkzeug gesteuert werden. Aus der ermittelten Position des Behandlungswerkzeuges lässt sich auch auf die Ist-Form zurückschließen, da sich an der Position des Behandlungswerkzeuges kein Zahn befindet. Beispielsweis kann mit einem Tastwerkzeug als Behandlungswerkzeug, dessen Position verfolgt wird, die dreidimensionale Ist-Zahnform berechnet werden.

In einer weiteren technisch vorteilhaften Ausführungsform des dentalen Führungssystems ist das Führungssystem ausgebildet, eine elektronische Datei zu erzeugen, die einen Ablauf einer Behandlung dokumentiert. Dadurch wird beispielsweise der technische Vorteil erreicht, dass Informationen über die Behandlung dauerhaft elektronisch gespeichert werden können.

In einer weiteren technisch vorteilhaften Ausführungsform des dentalen Führungssystems ist das Führungssystem ausgebildet, die räumliche Position der Datenbrille zu erfassen. Dadurch wird beispielsweise der technische Vorteil erreicht, dass die räumliche Position der Datenbrille bei der Ermittlung der dreidimensionalen Ist-Zahnform berücksichtigt werden kann. Dadurch wird die Genauigkeit der ermittelten Ist-Zahnform erhöht. Durch die Berücksichtigung eines Winkels kann die Genauigkeit einer Projektion der Abweichungsinformation in der Datenbrille erhöht werden.

In einer weiteren technisch vorteilhaften Ausführungsform des dentalen Führungssystems ist das Erfassungssystem ein optisches Erfassungssystem, das eine Autofocus- oder Zoomfunktion zum Erfassen der Ist-Zahnform umfasst. Die Autofocus-Funktion erlaubt eine automatische Scharfstellung, indem eine Kameraeinstellung an die Entfernung zwischen Kamera und Zahn angepasst wird und der Zahn scharf abgebildet wird. Die Zoom-Funktion erlaubt eine stufenlose Anpassung des Bildausschnitts an den Zahn. Dadurch wird beispielsweise der technische Vorteil erreicht, dass sich die Bedienung und Handhabung des Führungssystems vereinfacht.

In einer weiteren technisch vorteilhaften Ausführungsform des dentalen Führungssystems umfasst das Führungssystem einen Spiegel zum Erfassen der dreidimensionalen Ist-Zahnform. Durch den Spiegel wird beispielsweise der technische Vorteil erreicht, dass rückwärtige Bereiche des Zahnes räumlich erfasst werden können, die nicht direkt optisch zugänglich sind. Dadurch kann eine Rekonstruktion der Ist-Zahnform verbessert werden.

Ausführungsbeispiele der Erfindung sind in den Zeichnungen dargestellt und werden im Folgenden näher beschrieben.

Es zeigen:
- Fig. 1: eine schematische Ansicht eines dentalen Führungssystems; und
- Fig. 2: eine Datenbrille zur Betrachtung eines Zahnes.

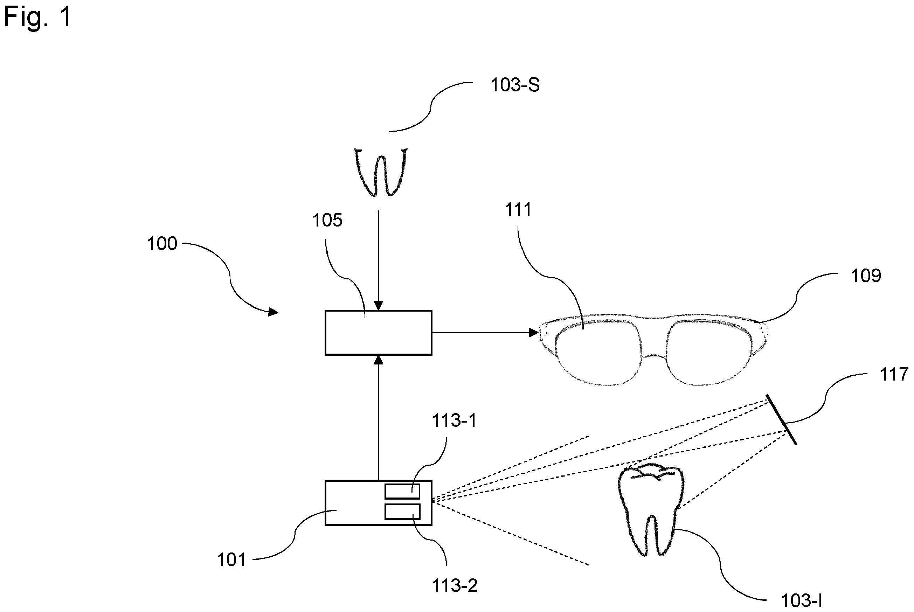

Fig. 1 zeigt eine schematische Ansicht eines dentalen Führungssystems 100. Das Führungssystem 100 dient zum Führen einer behandelnden Person, die eine Zahnpräparation im Mund eines Patienten durchführt. Mit dem dentalen Führungssystem 100 wird die manuelle Formgebung des Zahnes optisch unterstützt. Das dentale Führungssystem 100 ist nicht nur zur Formgebung von natürlichen Einzelzähnen geeignet, sondern kann ebenso zur Formgebung von künstlichen Zähnen oder dentalen Hilfsmitteln verwendet werden, wie beispielsweise Brücken, Teilprothesen, Implantaten, Bohrschablonen oder Abutments.

Das dentale Führungssystem 100 umfasst hierzu ein elektronisches Erfassungssystem 101, mit dem sich die dreidimensionale Ist-Zahnform 103-I im Mund des Patienten kontinuierlich während der Formgebung ermitteln lässt. Das Ermitteln der Ist-Zahnform 103-I kann auf optischem Wege erfolgen. Im Allgemeinen kann hierzu jedoch auch jedes andere Verfahren verwendet werden, mit dem sich die räumliche Ist-Zahnform 103-I ermitteln lässt. Das elektronische Erfassungssystem 101 erzeugt beispielsweise einen Datensatz, der die räumliche Form des Zahnes beschreibt.

Das Erfassungssystem 101 umfasst beispielsweise ein stereoskopisches Kamerasystem mit zwei elektronischen Kameras 113-1, 113-2. Durch die stereoskopische Aufnahme mit den beiden Kameras 113-1, 113-2 lässt sich die räumliche Ist-Zahnform 103-I auf einfache Weise ermitteln und rekonstruieren. Aus den jeweiligen Bildern der Kameras 113-1, 113-2 unter unterschiedlichen Parallaxenwinkeln, lässt sich auf die Ist-Zahnform 103-I mittels eines Computeralgorithmus berechnen.

Das Erfassungssystem 101 kann auch eine Kamera umfassen, die mit einem Laufzeitverfahren von Licht einzelne Distanzen ermittelt (TOF-Kamera - Time of Flight Kamera). Zu diesem Zweck wird der Zahn mittels eines Lichtpulses ausgeleuchtet und für jeden Bildpunkt die Zeit gemessen, die das Licht bis zum Objekt und wieder zurück braucht. Diese Zeit ist direkt proportional zur Distanz. Die Kamera liefert für jeden Bildpunkt die Entfernung des darauf abgebildeten Zahnes.

Das Erfassungssystem 101 kann die Ist-Zahnform 103-I auch auf einer Serie von Zahnbildern des Zahnes ermitteln, die aus unterschiedlichen Blickwinkeln gewonnen worden sind. Zu diesem Zweck kann ein Berechnungsverfahren verwendet werden, dass aus den einzelnen Zahnbildern die Ist-Zahnform 103-I rekonstruiert. Hierzu kann zusätzlich eine Information über die räumliche Position des Erfassungssystems 101 verwendet werden.

Das Erfassungssystem 101 kann jedoch auch auf einem Computertomographieverfahren, Röntgenverfahren oder einem Magnetresonanzverfahren basieren, um die Ist-Zahnform 103-I zu ermitteln. Dadurch wird der Vorteil erreicht, dass sich auch dreidimensionale Formen bestimmen lassen, die optisch nicht zugänglich sind, wie beispielsweise Zahnformen mit Läsionen. Durch diese Verfahren kann somit die Innensituation und das Innenvolumen des Zahnes erfasst werden. Umfasst beispielsweise der Zahn ein Loch, so kann eine entsprechende Ist-Zahnform erhalten werden, die nicht nur die äußere optisch wahrnehmbare Oberfläche wiedergibt. Die räumliche Form des Loches kann über die Anzeigeeinrichtung 111 angezeigt werden, so dass auch in diesem Fall das Erzeugen der Soll-Zahnform unterstützt werden kann. Bei minimalinvasiven Verfahren kann dadurch die Position des Bohrloches bestimmt werden.

Die ermittelte Ist-Zahnform 103-I wird einer elektronischen Vergleichseinrichtung 105 als Datensatz zugeführt. Die Vergleichseinrichtung 105 ermittelt die räumlichen Abweichungsbereiche 107, an denen die ermittelte Ist-Zahnform 103-I von einer vorgegebenen Soll-Zahnform 103-S abweicht.

Die Soll-Zahnform 103-S wird beispielsweise zuvor in einem Zwischenschritt über eine Benutzerschnittstelle ausgewählt oder automatisch an Hand von klinischen Fällen, beispielsweise aus einer Datenbank oder durch Algorithmen (Maschine Learning), vorgeschlagen oder speziell gestaltet, beispielsweise mit einem CAD-Verfahren, und der elektronischen Vergleichseinrichtung 105 ebenfalls als Datensatz zugeführt. Eine Datenbank kann eine Anzahl an Datensätzen für mögliche Soll-Zahnformen speichern. Diese Menge an Soll-Zahnformen kann entweder automatisch oder manuell ausgewählt werden. Zudem ist es möglich vorgegebene Soll-Zahnformen über eine Benutzerschnittstelle zu verändern.

Zum Ermitteln der Abweichung wird beispielsweise der Datensatz, der die räumliche Ist-Zahnform 103-I beschreibt, mit dem anderen Datensatz verglichen, der die Soll-Zahnform 103-S beschreibt. Stimmen beispielsweise Ist- und Soll-Zahnform 103-I, 103-S im Rahmen eines vorgegebenen Toleranzwertes nicht überein, wird der entsprechende Bereich als ein Abweichungsbereich 107 erfasst.

Die elektronische Vergleichseinrichtung 105 ist beispielsweise durch ein Software-Modul gebildet, das auf einer Computereinrichtung mit einem Prozessor und einem elektronischen Speicher zum Speichern des Softwaremoduls und der Datensätze ausgeführt wird.

Die so ermittelten räumlichen Abweichungsbereiche 107 werden an die Datenbrille 109 übermittelt, durch die die Zahnpräparation unterstützt wird. Die Datenbrille 109 hebt die Abweichungsbereiche 107 optisch hervor.

Die Datenbrille 109 (auch Augmented-Reality-Brille oder Smart-Glasses) ist ein tragbares Gerät, das in der Lage ist, virtuell Informationen vor die Augen des Brillenträgers zu projizieren, während dieser die Umwelt weiterhin visuell wahrnehmen kann. Dadurch können Informationen in dem Sichtfeld des Trägers angezeigt und hinzugefügt werden. Zu diesem Zweck umfasst die Datenbrille 109 eine Anzeigeeinrichtung 111, die aus einem augennahen Bildschirm oder einen Projektor zur direkten Projektion auf der Netzhaut gebildet sein kann.

Die Anzeigeeinrichtung 111 dient zum Anzeigen derjenigen Bereiche, an denen die ermittelte Ist-Zahnform 103-I von der vorgegebenen Soll-Zahnform 103-S abweicht. Auf diese Weise kann ein Träger der Datenbrille 109 unmittelbar auf dem visuell wahrgenommenen Zahn erkennen, welche räumlichen Bereiche von dem Zahn entfernt werden müssen, um die gewünschte Soll-Zahnform 103-S zu erhalten. Der Träger der Datenbrille 109 verwendet ein dentales Werkzeug, wie beispielsweise einen Bohrer oder Fräser, um die hervorgehobenen räumlichen Bereiche des Zahnes zu entfernen.

Die Datenbrille 109 kann zusätzlich noch Sensoren zur Bewegungserfassung des Kopfes oder zur räumlichen Positionserkennung der Datenbrille 109 umfassen, wie beispielsweise einen Gyrosensor. Damit kann die Anzeige der berechneten Abweichungsbereiche 107 genauer an die Bewegungen des Trägers angepasst werden. Die räumliche Position der Datenbrille 109 kann beispielsweise auf Basis vorgegebener optischer Referenzpunkte oder Marker erfolgen, die in der Umgebung der Datenbrille 109 angeordnet sind, wie beispielsweise mittels einer Trilateration oder einer Triangulation.

Das Erfassungssystem 101 kann in die Datenbrille 109 integriert sein. Dadurch ergibt sich der technische Vorteil, dass die Ist-Zahnform 103-I auf einfache Weise während dem Tragen der Datenbrille 109 durchgeführt werden kann. Im Allgemeinen kann das Erfassungssystem 101 auch als separates Gerät vorgesehen sein, mit dem die Ist-Zahnform 103-I während der Formgebung ermittelt wird.

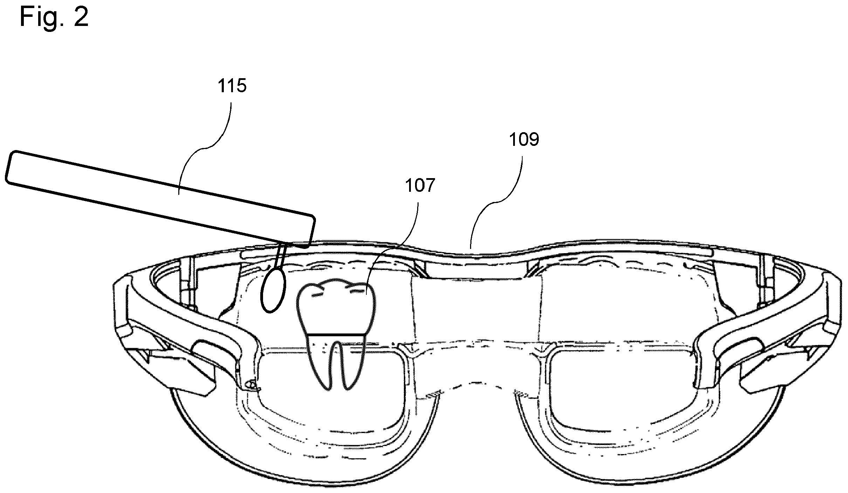

Fig. 2 zeigt die Datenbrille 109 zur Betrachtung des Zahnes und zum Anzeigen der Abweichungsbereiche 107. Die zu entfernenden Abweichungsbereiche 107 werden direkt über den visuell wahrnehmbaren Zahn überlagert. Dadurch kann der Träger der Datenbrille 109 bei der Zahnpräparation in Echtzeit erkennen, an welchen Stellen die Ist-Zahnform 103-I von der Sollzahnform 103-S abweicht.

Die Soll-Zahnform 103-S kann manuell auf Basis einer Bibliothek ausgewählt werden, die eine Menge an Soll-Zahnformen 103-S umfasst. Mögliche Soll-Zahnformen 103-S können dem Träger auf der Datenbrille 109 mittels der Anzeigeeinrichtung 111 angezeigt werden.

Allerdings ist es auch möglich, dass die Vergleichseinrichtung 105 die Soll-Zahnform 103-S auf Basis der zuvor erfassten Ist-Zahnform 103-I auswählt. In diesem Fall kann aus einer Menge an vorgegebenen Soll-Zahnformen 103-S diejenige ausgewählt werden, mit der im Vergleich zur Ist-Zahnform 103-I die meiste Zahnsubstanz erhalten bleibt.

Zudem kann die Vergleichseinrichtung 105 ausgebildet sein, dass diese einen Restaurationsparameter auf Basis der erfassten Ist-Zahnform 103-I automatisch ermittelt. Dieser kann beispielsweise ein Präparationswinkel sein, der angibt, um welchen Winkel die gegenwärtige Ist-Zahnform 103-I von einer Kronenlängsachse abweicht, d.h. beispielsweise ein halber Kegelwinkel. Zu diesem Zweck wird auf Basis der kontinuierlich erfassten Ist-Zahnform 103-I ermittelt, welchen Präparationswinkel diese aufweist.

Der Träger der Datenbrille 109 erhält den aktuellen Präparationswinkel angezeigt, so dass dieser den Zahn solange bearbeiten kann bis der richtige Präparationswinkel, d.h. der Winkel des präparierten Stumpfes, erreicht ist.

Im Allgemeinen kann der Restaurationsparameter jedoch auch andere Eigenschaften beschreiben, wie beispielsweise andere geometrische Parameter, die einen Zahnstumpf, ein Implantat, ein Abutment, Hinterschnitte, Kieferknochensituationen oder einen Einschubwinkel betreffen oder Parameter, die die ästhetische Situation betreffen, wie beispielsweise eine Farbe, einen Punkt auf dem Zahn, die Gingiva, Papille, Gaumenfalte oder das Zahnfleisch. Zusätzlich kann das elektronische Erfassungssystem 101 die Position eines Behandlungswerkzeuges 115 oder eines Spiegels 117 erfassen.

Weiter kann das Führungssystem 100 ausgebildet sein, die Formgebung des Zahnes mittels des Erfassungssystems 101 kontinuierlich zu erfassen und eine elektronische Datei zu erzeugen, die einen Ablauf einer Behandlung lückenlos dokumentiert, wie beispielsweise eine Videodatei oder CAD-Datei. In diesem Fall kann auch im Nachhinein die Formgebung des Zahnes auf Basis der elektronischen Datei verfolgt und begutachtet werden.

Das Erfassungssystem 101 kann zudem ausgebildet sein, zusätzlich zur Ist-Zahnform die Zahnfarbe des Zahnes optisch zu erfassen, beispielsweise indem digitale Farbwerte der Zahnfarbe während einer Aufnahme gewonnen werden. Diese Farbwerte können zusätzlich zur erfassten Ist-Zahnform in dem jeweiligen Datensatz gespeichert werden.

Das Führungssystem kann zudem einen Spiegel 117 zum Unterstützen des Erfassens der dreidimensionalen Ist-Zahnform aufweisen. Durch den Spiegel 117 wird die Erfassung der Ist-Zahnform beschleunigt, indem rückwärtige Bereiche des Zahnes räumlich erfasst werden können, die nicht direkt optisch zugänglich sind, wie beispielsweise eine Innenseite des Zahnes. Durch den Spiegel 117 lassen sich auf einfache und schnelle Weise zusätzliche Zahnbilder ermitteln, die zu einem (Teil-) Rundumbild des Zahnes zusammengesetzt werden können, das zur weiteren Rekonstruktion der Ist-Zahnform verwendet wird. Zum Ermitteln der Zahnform können im Allgemeinen geeignete Computeralgorithmen verwendet werden.

Der Spiegel 117 kann beispielsweise fest mit der optischen Erfassungseinrichtung 111 verbunden sein, so dass eine optische Erfassung gleichzeitig sowohl auf der Vorderseite als auch der Rückseite des Zahnes durchgeführt werden kann. Beispielsweise kann es sich um einen Spiegel mit integriertem Kamera-/Videosystem handeln.

Im Allgemeinen kann der Spiegel 117 jedoch auch ein in der Hand gehaltener Spiegel 117 sein, mit dem sich Zahnbilder der Rückseite des Zahnes erhalten lassen. Diese Zahnbilder können ebenfalls für eine Rekonstruktion der Ist-Zahnform herangezogen werden. Zudem kann durch die Erfassungseinrichtung 101 die Position des Spiegels 117 erfasst werden, so dass diese Position bei der Ermittlung der Ist-Zahnform berücksichtigt werden kann, wie beispielsweise mittels eines Ray-Tracing-Verfahrens.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Dentales Führungssystem
- 101: Erfassungssystem
- 103-I: Ist-Zahnform
- 103-S: Soll-Zahnform
- 105: Vergleichseinrichtung
- 107: Abweichungsbereich
- 109: Datenbrille
- 111: Anzeigeeinrichtung
- 113-1: Kamera
- 113-2: Kamera
- 115: Behandlungswerkzeug
- 117: Spiegel

## Patentansprüche

1. Dentales Führungssystem (100) zur Zahnpräparation, das ausgebildet ist, den Restaurationsparameter auf der Anzeigeeinrichtung (111) der Datenbrille (109) anzuzeigen, mit:
- einem elektronischen Erfassungssystem (101) zum Erfassen einer dreidimensionalen Ist-Zahnform (103-I), das ausgebildet ist, die dreidimensionale Ist-Zahnform (103-I) auf Basis einer zeitlichen Serie von Zahnbildern zu ermitteln und die Position eines Behandlungswerkzeuges (115) zu erfassen;
- einer Vergleichseinrichtung (105) zum Ermitteln von räumlichen Abweichungsbereichen (107), an denen die ermittelte Ist-Zahnform (103-I) von einer vorgegebenen Soll-Zahnform (103-S) abweicht und die ausgebildet ist, einen Restaurationsparameter auf Basis der erfassten Ist-Zahnform (103-I) zu ermitteln; und
- einer Datenbrille (109) mit einer Anzeigeeinrichtung (111) zum optischen Anzeigen der räumlichen Abweichungsbereiche (107), die das elektronische Erfassungssystem (101) umfasst.

2. Dentales Führungssystem (100) nach Anspruch 1, wobei das elektronische Erfassungssystem (101) ein stereoskopisches Erfassungssystem mit einer ersten und einer zweiten Kamera (113-1, 113-2) oder ein stereoskopisches Erfassungssystem mit einer plenoptischen Kamera umfasst.

3. Dentales Führungssystem (100) nach einem der vorangehenden Ansprüche, wobei das elektronische Erfassungssystem (101) eine 3D-Kamera zum Erfassen einer dreidimensionalen Ist-Zahnform (103-I) auf Basis einer Laufzeitmessung von Licht umfasst.

4. Dentales Führungssystem (100) nach einem der vorangehenden Ansprüche, wobei das Führungssystem (100) ausgebildet ist, eine elektronische Datei zu erzeugen, die einen Ablauf einer Behandlung dokumentiert.

5. Dentales Führungssystem (100) nach einem der vorangehenden Ansprüche, wobei das Führungssystem (100) ausgebildet ist, die räumliche Position der Datenbrille (109) zu erfassen.

6. Dentales Führungssystem (100) nach einem der vorangehenden Ansprüche, wobei das Erfassungssystem (111) ein optisches Erfassungssystem ist, das eine Autofocus- oder Zoomfunktion zum Erfassen der Ist-Zahnform umfasst.

7. Dentales Führungssystem (100) nach einem der vorangehenden Ansprüche, wobei das Führungssystem (100) einen Spiegel (117) zum Erfassen der dreidimensionalen Ist-Zahnform (103-I) umfasst.

## Claims

1. Dental guidance system (100) for tooth preparation, which is adapted to display the restoration parameter on the display device (111) of the data glasses (109), comprising:
- an electronic detection system (101) for detecting a three-dimensional actual tooth shape (103-1), which is adapted to determine the three-dimensional actual tooth shape (103-1) on the basis of a time series of tooth images and to detect the position of a treatment tool (115) ;
- a comparison means (105) for determining spatial deviation regions (107) at which the determined actual tooth form (103-1) deviates from a predetermined desired tooth form (103-S) and which is designed to determine a restoration parameter on the basis of the detected actual tooth form (103-1); and
- data glasses (109) having display means (111) for optically displaying the spatial deviation regions (107), comprising the electronic detection system (101).

2. Dental guidance system (100) according to claim 1, wherein said electronic detection system (101) comprises a stereoscopic detection system with a first and a second camera (113-1, 113-2) or a stereoscopic detection system with a plenoptic camera.

3. Dental guidance system (100) according to any one of the preceding claims, wherein the electronic acquisition system (101) comprises a 3D camera for acquiring a three-dimensional actual tooth shape (103-1) based on a time-of-flight measurement of light.

4. Dental guidance system (100) according to any one of the preceding claims, wherein the guidance system (100) is adapted to generate an electronic file documenting a course of treatment.

5. Dental guidance system (100) according to any one of the preceding claims, wherein the guidance system (100) is configured to detect the spatial position of the data glasses (109).

6. Dental guidance system (100) according to any one of the preceding claims, wherein the detection system (101) is an optical detection system comprising an autofocus or zoom function for detecting the actual tooth shape.

7. Dental guidance system (100) according to any one of the preceding claims, wherein the guidance system (100) comprises a mirror (117) for detecting the three-dimensional actual tooth shape (103-1).

## Revendications

1. Système de guidage dentaire (100) pour la préparation des dents adapté pour afficher le paramètre de restauration sur le dispositif d'affichage (111) des lunettes de données (109), comprenant :
- un système de détection électronique (101) pour détecter une forme de dent réelle tridimensionnelle (103-I), qui est adapté pour déterminer la forme de dent réelle tridimensionnelle (103-I) sur la base d'une série temporelle d'images de dents et pour détecter la position d'un outil de traitement (115) ;
- un dispositif de comparaison (105) pour déterminer des zones d'écart spatial (107) dans lesquelles la forme de dent réelle déterminée (103-I) s'écarte d'une forme de dent prescrite prédéterminée (103-S) et qui est adapté pour déterminer un paramètre de restauration sur la base de la forme de dent réelle détectée (103-I); et
- lunettes de données (109) ayant un dispositif d'affichage (111) pour afficher optiquement les régions de déviation spatiale (107), comprenant le système de détection électronique (101).

2. Système de guidage dentaire (100) selon la revendication 1, dans lequel le système de détection électronique (101) comprend un système de détection stéréoscopique avec une première et une deuxième caméra (113-1, 113-2) ou un système de détection stéréoscopique avec une caméra plénoptique.

3. Système de guidage dentaire (100) selon l'une quelconque des revendications précédentes, dans lequel le système de détection électronique (101) comprend une caméra 3D pour détecter une forme de dent réelle tridimensionnelle (103-I) sur la base d'une mesure de temps de vol de la lumière.

4. Système de guidage dentaire (100) selon l'une quelconque des revendications précédentes, dans lequel le système de guidage (100) est adapté pour générer un fichier électronique documentant un cours de traitement.

5. Système de guidage dentaire (100) selon l'une quelconque des revendications précédentes, dans lequel le système de guidage (100) est adapté pour détecter la position spatiale des lunettes de données (109).

6. Système de guidage dentaire (100) selon l'une quelconque des revendications précédentes, dans lequel le système de détection (111) est un système de détection optique comprenant une fonction autofocus ou zoom pour détecter la forme réelle de la dent.

7. Système de guidage dentaire (100) selon l'une quelconque des revendications précédentes, dans lequel le système de guidage (100) comprend un miroir (117) pour détecter la forme réelle tridimensionnelle de la dent (103-1).
